# EUROPEAN PATENT SPECIFICATION

(11) **EP 3 520 788 B1**
(45) Date of publication and mention of the grant of the patent: **11.02.2026**
(21) Application number: 19165074.6
(22) Date of filing: 05.09.2012
(51) Int. Cl.: A61K 31/4152, A61P 11/16, A61P 21/02, A61P 21/00, A61P 21/04, A61P 25/00, A61P 25/28, C07D 231/26

(54) **EDARAVONE FOR TREATING AMYOTROPHIC LATERAL SCLEROSIS OR PREVENTING PROGRESSION OF PHASE OF AMYOTROPHIC LATERAL SCLEROSIS**
EDARAVONE ZUR BEHANDLUNG VON AMYOTROPHER LATERALSKLEROSE ODER VERHINDERUNG DES FORTSCHREITENS VON AMYOTROPHER LATERALSKLEROSE
EDARAVONE POUR LE TRAITEMENT DE LA SCLÉROSE LATÉRALE AMYOTROPHIQUE OU LA PRÉVENTION DE L'ÉVOLUTION D'UNE PHASE DE LA SCLÉROSE LATÉRALE AMYOTROPHIQUE

(30) Priority: 05.09.2011 JP 2011192288
(43) Date of publication of application: 07.08.2019
(62) Divisional of application: 12830484.7
(73) Proprietor: Mitsubishi Tanabe Pharma Corporation, Osaka-shi Osaka 541-8505 (JP)
(72) Inventor: YONEOKA, Takatomo, Osaka 541-8505 (JP)
(74) Representative: Hoffmann Eitle

(56) References cited:
- EP-A1- 1 714 960
- YOSHINO HIIDE ET AL: "Investigation of the therapeutic effects of edaravone, a free radical scavenger, on amyotrophic lateral sclerosis (Phase II study)", AMYOTROPHIC LATERAL SCLER, UNITED KINGDOM, vol. 7, no. 4, 1 December 2006 (2006-12-01), pages 241 - 245, XP009134282, ISSN: 1748-2968
- BRYAN J. TRAYNOR ET AL: "Clinical Features of Amyotrophic Lateral Sclerosis According to the El Escorial and Airlie House Diagnostic Criteria", ARCHIVES OF NEUROLOGY, vol. 57, no. 8, 1 August 2000 (2000-08-01), pages 1171, XP055146043, ISSN: 0003-9942, DOI: 10.1001/archneur.57.8.1171
- JESSE M. CEDARBAUM ET AL: "The ALSFRS-R: a revised ALS functional rating scale that incorporates assessments of respiratory function", JOURNAL OF NEUROLOGICAL SCIENCES., vol. 169, no. 1-2, 1 October 1999 (1999-10-01), NL, pages 13 - 21, XP055289508, ISSN: 0022-510X, DOI: 10.1016/S0022-510X(99)00210-5
- UNANYMOUS: "A post-hoc subgroup analysis of outcomes in the first phase III clinical study of edaravone (MCI-186) in amyotrophic lateral sclerosis", 5 September 2017 (2017-09-05), XP093193674, Retrieved from the Internet <URL:https://www.tandfonline.com/doi/full/10.1080/21678421.2017.1363780>

## Description

### Technical Field

The present invention relates to a pharmaceutical agent for use in treating amyotrophic lateral sclerosis (hereinafter also referred to as "ALS") or suppressing the disease progress thereof, or treating symptoms caused by ALS or suppressing the disease progress thereof as defined in the present claims.

### Background Art

ALS, which is one type of motor neuron disease, is intractable disease. ALS starts with initial symptoms such as weakness in hands, movement disorders with fingers and fascicular contraction in upper limbs. Thereafter, ALS has amyotrophia and/or muscular weakness, bulbar paralysis and fascicular contraction in muscles, and it finally leads to respiratory failure. ALS is divided into upper limb, bulbar, lower limb and mixed types, depending on the site of onset. In all of the aforementioned types, as symptoms progress, a systemic muscle group is affected. The causal factor of ALS has not yet been sufficiently elucidated. The following hypothesis has been proposed as main causal factors of ALS: (1) autoimmunity (the appearance of an autoantibody against the Ca channel); (2) excessive excitatory amino acid and/or toxication (an increase in extracellular glutamic acid and glutamic acid transport disorders); (3) oxidative stress disorders (Cu/Zn superoxide dismutase (SOD) genetic abnormality and neuronopathy caused by free radicals); (4) cytoskeleton disorders (accumulation of neurofilament in motor nerve cells and the appearance of inclusion bodies); and (5) the absence of neurotrophic factors.

At present, as a pharmaceutical product effective for suppression of the progress of ALS, there has been approved "riluzole," which suppresses glutamic acid transport in the glutamatergic nerve. However, it has been reported that the effectiveness of riluzole could not be confirmed. Thus, the evaluation of this drug is not stable.

Moreover, an ALS therapeutic agent comprising 3-methyl-1-phenyl-2-pyrazolin-5-one has been known (Patent Literature 1). Patent Literature 1 suggests or teaches the usefulness of 3-methyl-1-phenyl-2-pyrazolin-5-one for the treatment of ALS. However, this document does not specifically disclose the administration form, the applied dose, the number of doses, and the like of this compound to patients.

Furthermore, there has also been known an agent for treating amyotrophic lateral sclerosis or symptoms caused by amyotrophic lateral sclerosis, and/or for suppressing the progress thereof, which comprises 3-methyl-1-phenyl-2-pyrazolin-5-one as an active ingredient, wherein a drug holidays period for 1 or more days is established one or two or more times, in a period in which the disease is treated and/or the progress thereof is suppressed (Patent Literature 2). Furthermore, there has also been reported a method of administering 30 mg of 3-methyl-1-phenyl-2-pyrazolin-5-one to an ALS patient by infusion for 14 days, and then administering it for 10 days in 1 month (Non Patent Literature 1). Non Patent Literature 2 discloses edaravone for use in treating ALS by administering the compound intravenously for 14 consecutive days. After an observation period of two weeks, the four-week cycle was repeated six times.

### [Prior Art Literatures]

### [Patent Literature]

[Patent Literature 1] International Publication WO02/34264
[Patent Literature 2] International Publication WO2005/075434

### [Non Patent Literature]

[Non Patent Literature 1] Shinkei Chiryo (Neurotherapeutics), 2003, Vol. 20, No. 5, pp. 557-564
[Non Patent Literature 2] Yoshino et al., Amyotrophic lateral sclerosis, vol. 7, no. 4, 2006

### [Disclosure of Invention]

### [Problem to be Solved by the Invention]

In general, in order to obtain approval for the addition of efficacy to a pharmaceutical product, the effects (effectiveness) thereof need to be confirmed in a novel clinical test. The effects of a pharmaceutical product may be different depending on the conditions of patients. Thus, it may be preferable that patients in whom the effects of a pharmaceutical product are sufficiently observed be selected, and that the pharmaceutical product be then administered to the thus selected patients.

It is an object of the present invention to provide a novel pharmaceutical agent for treating ALS or suppressing the disease progress thereof, or treating symptoms caused by ALS or suppressing the disease progress thereof, wherein the aforementioned agent can be administered particularly to patients who are able to obtain high therapeutic effects, among ALS patients who require treatments. In addition, explaining the object of the present invention from another viewpoint, it is another object of the present invention to provide a novel pharmaceutical agent or method for treating specific ALS or suppressing the disease progress thereof, or treating symptoms caused by such specific ALS or suppressing the disease progress thereof.

### Means for Solving the Problem

As a result of intensive studies directed towards achieving the aforementioned object, the present inventor has found that a pharmaceutical composition, which has been placed on the market since June, 2001, as a cerebroprotective agent (common name: "edaravone," trade name: "Radicut (registered trademark)," manufactured and distributed by Mitsubishi Tanabe Pharma Corporation), is particularly effective for a specific group of patients with ALS, among ALS patients, thereby completing the present invention.

The present invention provides a pharmaceutical agent for use in treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein
the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
a patient administered with the agent is a patient [1] who scores two or more points from all items constituting the ALSFRS-R; and [2] whose %FVC is 80% or more, and
   wherein
the applied dose of 3-methyl-1-phenyl-2-pyrazolin-5-one or a physiologically acceptable salt thereof per day corresponds to an amount of 15 mg to 240 mg 3-methyl-1-phenyl-2-pyrazolin-5-one.

The present disclosure also relates to a patient administered with the agent being any one of the following [1] to [4]:
[1] a patient who is determined to be "Definite ALS" or "Probable ALS" according to the revised El Escorial (Airlie House) diagnostic criteria;
[2] a patient who is determined to be "Definite ALS" according to the revised El Escorial (Airlie House)the revised El Escorial (Airlie House) diagnostic criteria;
[3] a patient who is determined to be "Definite ALS" or "Probable ALS" according to the revised El Escorial (Airlie House)the revised El Escorial (Airlie House) diagnostic criteria, scores two or more points from all items constituting the ALSFRS-R, and whose %FVC is 80% or more; and
[4] a patient who is determined to be "Definite ALS" according to the revised El Escorial (Airlie House)the revised El Escorial (Airlie House) diagnostic criteria, scores two or more points from all items constituting the ALSFRS-R, and whose %FVC is 80% or more.

The present disclosure provides the following.
(1) A pharmaceutical agent for use in treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein
   the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
   a patient administered with the agent is a patient who is determined to be "Definite ALS" or "Probable ALS" according to the revised El Escorial (Airlie House) the revised El Escorial (Airlie House) diagnostic criteria.
(2) A pharmaceutical agent for use in treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein
   the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
   a patient administered with the agent is a patient who is determined to be "Definite ALS" according to the revised El Escorial (Airlie House)the revised El Escorial (Airlie House) diagnostic criteria.
(3) A pharmaceutical agent for treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein
   the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
   a patient administered with the agent is a patient who scores two or more points from all items constituting the ALSFRS-R.
(4) A pharmaceutical agent for treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein
   the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
   a patient administered with the agent is a patient whose %FVC is 80% or more.

The present invention further relates to:
(5) A pharmaceutical agent for use in treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein
the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
the agent is administered to a patient with amyotrophic lateral sclerosis, and the amyotrophic lateral sclerosis of the patient administered with the agent is amyotrophic lateral sclerosis [1] which scores two or more points from all items constituting the ALSFRS-R; and [2] whose %FVC is 80% or more and
   wherein
the applied dose of 3-methyl-1-phenyl-2-pyrazolin-5-one or a physiologically acceptable salt thereof per day corresponds to an amount of 15 mg to 240 mg 3-methyl-1-phenyl-2-pyrazolin-5-one.

The present disclosure further relates to:
(6) A pharmaceutical agent for use in treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein
   the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
   a patient administered with the agent is a patient who is determined to be "Definite ALS" or "Probable ALS" according to the revised El Escorial (Airlie House) diagnostic criteria, scores two or more points from all items constituting the ALSFRS-R, and whose %FVC is 80% or more.
(7) A pharmaceutical agent for use in treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein
   the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
   a patient administered with the agent is a patient who is determined to be "Definite ALS" according to the revised El Escorial (Airlie House) diagnostic criteria, scores two or more points from all items constituting the ALSFRS-R, and whose %FVC is 80% or more.

Preferably, with regard to the administration period and the drug holiday period, an initial 14-day administration period and an initial 14-day drug holiday period are established, and thereafter, an administration period for 10 out of 14 days and a 14-day drug holiday period are repeated.

Preferably, the symptoms caused by amyotrophic lateral sclerosis are a decrease in respiratory function, spoken language disorder, dysphagia, or limb movement disorder.

The present disclosure further provides 3-methyl-1-phenyl-2-pyrazolin-5-one or a physiologically acceptable salt thereof for use in treating amyotrophic lateral sclerosis or suppressing the disease progress thereof, or for use in treating symptoms caused by amyotrophic lateral sclerosis or suppressing the disease progress thereof, which comprises administering an effective amount of 3-methyl-1-phenyl-2-pyrazolin-5-one or a physiologically acceptable salt thereof to a patient, wherein
the administration is carried out by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
the patient is any one of the following [1] to [7]:
   [1] a patient who is determined to be "Definite ALS" or "Probable ALS" according to the revised El Escorial (Airlie House) diagnostic criteria;
   [2] a patient who is determined to be "Definite ALS" according to the revised El Escorial (Airlie House) diagnostic criteria;
   [3] a patient who scores two or more points from all items constituting the ALSFRS-R;
   [4] a patient whose %FVC is 80% or more;
   [5] a patient who scores two or more points from all items constituting the ALSFRS-R and whose %FVC is 80% or more;
   [6] a patient who is determined to be "Definite ALS" or "Probable ALS" according to the revised El Escorial (Airlie House) diagnostic criteria, scores two or more points from all items constituting the ALSFRS-R, and whose %FVC is 80% or more; and
   [7] a patient who is determined to be "Definite ALS" according to the revised El Escorial (Airlie House) diagnostic criteria, scores two or more points from all items constituting the ALSFRS-R, and whose %FVC is 80% or more.

The present disclosure further provides 3-methyl-1-phenyl-2-pyrazolin-5-one or a physiologically acceptable salt thereof for use in treating amyotrophic lateral sclerosis or suppressing the disease progress thereof, or treating symptoms caused by amyotrophic lateral sclerosis or suppressing the disease progress thereof, which comprises a step of selecting any one of the following patients [1] to [7] from patients with amyotrophic lateral sclerosis and a step of administering an effective amount of 3-methyl-1-phenyl-2-pyrazolin-5-one or a physiologically acceptable salt thereof to the thus selected patient, wherein
a 14-day administration period and a 14-day drug holiday period are repeated, or an initial 14-day administration period and an initial 14-day drug holiday period are established, and thereafter, an administration period for 10 out of 14 days and a 14-day drug holiday period are repeated:
[1] a patient who is determined to be "Definite ALS" or "Probable ALS" according to the revised El Escorial (Airlie House) diagnostic criteria;
[2] a patient who is determined to be "Definite ALS" according to the revised El Escorial (Airlie House) diagnostic criteria;
[3] a patient who scores two or more points from all items constituting the ALSFRS-R;
[4] a patient whose %FVC is 80% or more %FVC;
[5] a patient who scores two or more points from all items constituting the ALSFRS-R and whose %FVC is 80% or more;
[6] a patient who is determined to be "Definite ALS" or "Probable ALS" according to the revised El Escorial (Airlie House) diagnostic criteria, scores two or more points from all items constituting the ALSFRS-R, and whose %FVC is 80% or more; and
[7] a patient who is determined to be "Definite ALS" according to the revised El Escorial (Airlie House) diagnostic criteria, scores two or more points from all items constituting the ALSFRS-R, and whose %FVC is 80% or more.

The present disclosure further provides an use of 3-methyl-1-phenyl-2-pyrazolin-5-one or a physiologically acceptable salt thereof for production of a pharmaceutical agent for treating amyotrophic lateral sclerosis or suppressing the disease progress thereof or treating symptoms caused by amyotrophic lateral sclerosis or suppressing the disease progress thereof, wherein
the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
a patient administered with the agent is any one of the following [1] to [7]:
   [1] a patient who is determined to be "Definite ALS" or "Probable ALS" according to the revised El Escorial (Airlie House) diagnostic criteria;
   [2] a patient who is determined to be "Definite ALS" according to the revised El Escorial (Airlie House) diagnostic criteria;
   [3] a patient who scores two or more points from all items constituting the ALSFRS-R;
   [4] a patient whose %FVC is 80% or more;
   [5] a patient who scores two or more points from all items constituting the ALSFRS-R and whose %FVC is 80% or more;
   [6] a patient who is determined to be "Definite ALS" or "Probable ALS" according to the revised El Escorial (Airlie House) diagnostic criteria, scores two or more points from all items constituting the ALSFRS-R, and whose %FVC is 80% or more; and
   [7] a patient who is determined to be "Definite ALS" according to the revised El Escorial (Airlie House) diagnostic criteria, scores two or more points from all items constituting the ALSFRS-R, and whose %FVC is 80% or more.

The present disclosure further provides a pharmaceutical agent for use in treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein
the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein
the agent is administered to an patient with amyotrophic lateral sclerosis, and the amyotrophic lateral sclerosis of the patient administered with the agent is any one of the following [1] to [7]:
   [1] amyotrophic lateral sclerosis, which complies with "Definite ALS" or "Probable ALS" according to the revised El Escorial (Airlie House) diagnostic criteria;
   [2] amyotrophic lateral sclerosis, which complies with "Definite ALS" according to the revised El Escorial (Airlie House) diagnostic criteria the revised El Escorial (Airlie House) diagnostic criteria;
   [3] amyotrophic lateral sclerosis, which scores two or more points from all items constituting the ALSFRS-R;
   [4] amyotrophic lateral sclerosis, a patient of which has 80% %FVC or more;
   [5] amyotrophic lateral sclerosis, which scores two or more points from all items constituting the ALSFRS-R and, a patient of which has 80% %FVC or more;
   [6] amyotrophic lateral sclerosis, which complies with "Definite ALS" or "Probable ALS" according to the revised El Escorial (Airlie House) diagnostic criteria, scores two or more points from all items constituting the ALSFRS-R and, a patient of which has 80% %FVC or more; and
   [7] amyotrophic lateral sclerosis, which complies with "Definite ALS" according to the revised El Escorial (Airlie House) diagnostic criteria, scores two or more points from all items constituting the ALSFRS-R and, a patient of which has 80% %FVC or more.

### Advantageous Effects of Invention

The pharmaceutical agent and uses of the present invention are useful for treating ALS or symptoms caused by ALS or for suppressing the disease progress thereof. According to the pharmaceutical agent and uses of the present invention, it is possible to reduce the number of doses or the number of hospital visits. Moreover, according to the pharmaceutical agent and uses of the present invention, the present agent can be administered particularly to a specific group of ALS patients who are able to obtain high therapeutic effects or high effects of suppressing disease progress, among ALS patients. Furthermore, according to the pharmaceutical agent and uses of the present invention, high therapeutic effects or high effects of suppressing disease progress can be provided on specific ALS among various types of ALS, or high therapeutic effects or high effects of suppressing disease progress can be provided on symptoms caused by such specific ALS.

### Embodiments of Carrying out the Invention

Hereinafter, the embodiments of the present invention will be described in detail.

The active ingredient of the pharmaceutical agent of the present invention is 3-methyl-1-phenyl-2-pyrazolin-5-one. 3-Methyl-1-phenyl-2-pyrazolin-5-one can be represented by the following structural formulae. The 3-methyl-1-phenyl-2-pyrazolin-5-one includes tautomers represented by the following structural formulae. Either one of these tautomers may be used as an active ingredient of the pharmaceutical agent of the present invention.

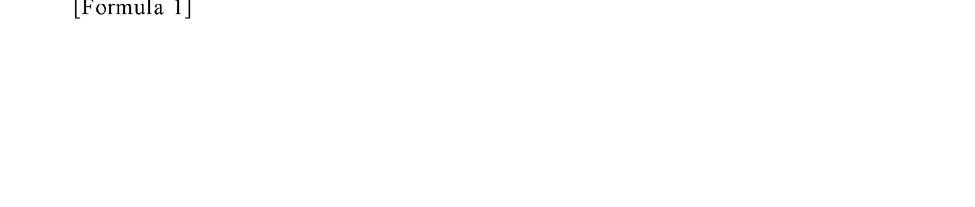

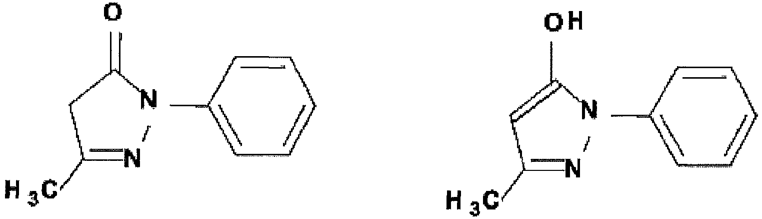

3-Methyl-1-phenyl-2-pyrazolin-5-one used as an active ingredient in the present invention is a known compound, and it can be synthesized by any given purposeful method. An example of the preferred synthetic method is a production method described in European Patent Application No. 208874 (or JP Patent Publication (Kokoku) No. 5-31523 B).

As an active ingredient of the pharmaceutical agent of the present invention, 3-methyl-1-phenyl-2-pyrazolin-5-one, a physiologically acceptable salt thereof, a hydrate thereof, or a solvate thereof may be used. Examples of the physiologically acceptable salt include: salts with mineral acids such as hydrochloric acid, sulfuric acid, hydrobromate or phosphoric acid; salts with organic acids such as methanesulfonic acid, p-toluenesulfonic acid, acetic acid, oxalic acid, citric acid, malic acid or fumaric acid; salts with alkaline metals such as sodium or potassium; salts with alkaline-earth metals such as magnesium; and salts with amines such as ammonia, ethanolamine or 2-amino-2-methyl-1-propanol. In addition to these salts, the types of salts are not particularly limited, as long as they are physiologically acceptable salts.

3-Methyl-1-phenyl-2-pyrazolin-5-one used as an active ingredient of the pharmaceutical agent of the present invention or a salt thereof may be directly administered to a patient. Preferably, pharmacologically and pharmaceutically acceptable additives are added to the active ingredient, and thus, the active ingredient or a salt thereof can be provided in the form of a pharmaceutical preparation that is well known to persons skilled in the art.

Examples of the pharmacologically and pharmaceutically acceptable additives that can be used herein include excipients, disintegrators or disintegration aids, binders, lubricants, coating agents, pigments, diluents, bases, solubilizers or solubilizing agents, isotonizing agents, pH adjusters, stabilizers, propellants, and adhesives. Examples of a pharmaceutical preparation suitable for oral administration include tablets, capsules, powder agents, fine grain agents, granules, liquid agents, and syrup agents. Examples of a pharmaceutical preparation suitable for parenteral administration include injections, drops, patches, and suppositories.

Examples of additives used for pharmaceutical preparations suitable for oral administration that can be used herein include: excipients such as glucose, lactose, D-mannitol, starch or crystalline cellulose; disintegrators or disintegration aids, such as carboxymethylcellulose, starch or carboxymethylcellulose calcium; binders such as hydroxypropylcellulose, hydroxypropylmethylcellulose, polyvinylpyrrolidone or gelatin; lubricants such as magnesium stearate or talc; coating agents such as hydroxypropylmethylcellulose, saccharose, polyethylene glycol or titanium oxide; and bases such as Vaseline, liquid paraffin, polyethylene glycol, gelatin, kaoline, glycerin, purified water or hard fat.

Examples of additives used for pharmaceutical preparations suitable for injections or drops that can be used herein include: solubilizers or solubilizing agents that can constitute aqueous injections or injections which are dissolved before use, such as distilled water for injections, a normal saline or propylene glycol; isotonizing agents such as glucose, sodium chloride, D-mannitol or glycerin; and pH adjusters such as inorganic acids, organic acids, inorganic bases or organic bases.

Since a cerebroprotective agent (injection) comprising 3-methyl-1-phenyl-2-pyrazolin-5-one as an active ingredient has already been used in clinical sites (common name: "edaravone," trade name: "Radicut (registered trademark)," manufactured and distributed by Mitsubishi Tanabe Pharma Corporation), this commonly available agent can be directly used as 3-methyl-1-phenyl-2-pyrazolin-5-one in the pharmaceutical agent and method of the present invention.

In general, when a certain disease is found in a human body, a treatment may be appropriately carried out by a doctor. A drug treatment is one of such treatments. In such a drug treatment, a drug is generally continuously administered to a patient until the patient recovers from the disease. In contrast, in the pharmaceutical agent and use of the present invention, a 14-day drug holiday period is established two or more times during a drug treatment period. That is to say, the pharmaceutical agent and use of the present invention are characterized in that a unit consisting of an administration period and a drug holiday period is repeated two or more times. Herein, when such an administration period and a drug holiday period are repeated two or more times, the final period should necessarily be a drug holiday period. However, it is not essential to establish such a final drug holiday period. That is, when an administration period and a drug holiday period are repeated two times, for example, it results in "an administration period, a drug holiday period, an administration period, and a drug holiday period." However, administration excluding the final drug holiday period, namely, administration consisting of "an administration period, a drug holiday period, and an administration period" is also included in the present invention. Further, the term "drug holiday period" is used in the present invention to mean a period in which drug administration cannot be carried out for continuous 7 or more days.

The administration period can be set at 14 days, or 10 out of 14 days in the present invention. The term "10 out of 14 days" is used herein to mean any given 10 days in continuous 14 days. The 10 days, in which a drug is administered, may be either continuous 10 days, or discontinuous 10 days disrupted with a period for 1 to 4 days in which the drug is not administered. A preferred administration period can be selected by observing patient's conditions.

The drug holiday period is 14 days in the present invention.

The number of repetitions in the case of repeating a 14-day administration period and a 14-day drug holiday period is not particularly limited, as long as it is two or more. It may be set at preferably 2 to 12, and more preferably 2 to 6.

The number of repetitions, in which an administration period for 10 out of 14 days and a 14-day drug holiday period are repeated in a case where an initial 14-day administration period and an initial 14-day drug holiday period are established, and thereafter, an administration period for 10 out of 14 days and a 14-day drug holiday period are repeated, is not particularly limited, as long as it is one or more. It may be set at preferably 1 to 11, and more preferably 1 to 5.

In general, the amount of 3-methyl-1-phenyl-2-pyrazolin-5-one applied to an adult (wherein it is the amount of 3-methyl-1-phenyl-2-pyrazolin-5-one if the active ingredient is 3-methyl-1-phenyl-2-pyrazolin-5-one, and it is an amount corresponding to 3-methyl-1-phenyl-2-pyrazolin-5-one if the active ingredient is a physiologically acceptable salt thereof) is approximately 15 mg to approximately 240 mg, preferably approximately 30 mg to approximately 60 mg, and more preferably approximately 60 mg.

The number of doses per day during the administration period is not limited, and a preferred number of doses can be selected while observing patient's conditions. However, taking into consideration a burden on the patient, the number of doses per day is preferably 3 times, 2 times or 1 time, and more preferably 1 time.

Upon administration of the active ingredient, the administration route is not particularly limited. The active ingredient can be administered orally or parenterally. Moreover, bolus administration and continuous administration are possible, and continuous administration is preferable. In the case of such continuous administration, intravenous administration involving drops, transdermal administration, oral administration using sublingual tablets, and oral and rectal administration using sustained-release agents can be applied. Among others, intravenous administration involving drops is preferable. When bolus administration involving injections or intravenous administration involving drops is carried out, it is preferable to use the injections described, for example, in JP Patent Publication (Kokai) No. 63-132833 A and JP Patent Publication (Kokai) No. 2011-62529 A, etc.

In the case of intravenous administration involving drops, the administration rate is desirably set at approximately 0.5 to approximately 1 mg/min, in terms of the amount of 3-methyl-1-phenyl-2-pyrazolin-5-one. If it is converted to the amount of time, it is approximately 15 to approximately 480 minutes, preferably approximately 30 to approximately 120 minutes, more preferably approximately 30 to approximately 60 minutes, and even more preferably approximately 60 minutes.

An administration form that is substantially equivalent to intravenous administration involving drops, in which the dose of 3-methyl-1-phenyl-2-pyrazolin-5-one per minute is set at approximately 0.5 mg to approximately 1 mg, may mean an administration form that is pharmacokinetically, substantially equivalent to the aforementioned intravenous administration. As a specific example, it is an administration form, in which a change over time in the unchanged drug concentration of 3-methyl-1-phenyl-2-pyrazolin-5-one or a physiologically acceptable salt thereof in plasma, which has been administered to a patient, is considered to be substantially equivalent to that in the aforementioned intravenous administration. Examples of such an administration form include transdermal administration, oral administration using sublingual tablets, and oral and rectal administration using sustained-release agents.

Examples of the symptoms caused by ALS include clinical symptoms such as a decrease in respiratory function, spoken language disorder, dysphagia, or limb movement disorder. In the present invention, a preferred example of the symptoms caused by ALS is a decrease in respiratory function. This term should be interpreted in the broadest sense, as long as it complies with the above-mentioned definition. It should not be interpreted with only a difference in disease names. It is to be noted that whether or not the concerned disease is considered to be ALS can be readily diagnosed by an experienced doctor.

Moreover, a preferred example of the treatment of ALS or the symptoms caused by ALS and/or suppression of the progress thereof can be suppression of a decrease in respiratory function in amyotrophic lateral sclerosis.

The pharmaceutical agent or use of the present disclosure is characterized in that a patient administered with the pharmaceutical agent is any one of the following [1] to [7]:
[1] a patient who is determined to be "Definite ALS" or "Probable ALS" according to the revised El Escorial (Airlie House) diagnostic criteria;
[2] a patient who is determined to be "Definite ALS" according to the revised El Escorial (Airlie House) diagnostic criteria;
[3] a patient who scores two or more points from all items constituting the ALSFRS-R;
[4] a patient whose %FVC is 80% or more;
[5] a patient who scores two or more points from all items constituting the ALSFRS-R and whose %FVC is 80% or more;
[6] a patient who is determined to be "Definite ALS" or "Probable ALS" according to the revised El Escorial (Airlie House) diagnostic criteria, scores two or more points from all items constituting the ALSFRS-R, and whose %FVC is 80% or more; and
[7] a patient who is determined to be "Definite ALS" according to the revised El Escorial (Airlie House) diagnostic criteria, scores two or more points from all items constituting the ALSFRS-R, and whose %FVC is 80% or more.

Furthermore, amyotrophic lateral sclerosis in a patient, to whom the pharmaceutical agent or use of the present disclosure is applied, is characterized in that it is any one of the following [1] to [7]:
[1] amyotrophic lateral sclerosis, which complies with "Definite ALS" or "Probable ALS" according to the revised El Escorial (Airlie House) diagnostic criteria;
[2] amyotrophic lateral sclerosis, which complies with "Definite ALS" according to the revised El Escorial (Airlie House) diagnostic criteria the revised El Escorial (Airlie House) diagnostic criteria;
[3] amyotrophic lateral sclerosis, which scores two or more points from all items constituting the ALSFRS-R;
[4] amyotrophic lateral sclerosis, a patient of which has 80% %FVC or more;
[5] amyotrophic lateral sclerosis, which scores two or more points from all items constituting the ALSFRS-R and, a patient of which has 80% %FVC or more;
[6] amyotrophic lateral sclerosis, which complies with "Definite ALS" or "Probable ALS" according to the revised El Escorial (Airlie House) diagnostic criteria, scores two or more points from all items constituting the ALSFRS-R and, a patient of which has 80% %FVC or more; and
[7] amyotrophic lateral sclerosis, which complies with "Definite ALS" according to the revised El Escorial (Airlie House) diagnostic criteria, scores two or more points from all items constituting the ALSFRS-R and, a patient of which has 80% %VFC or more.

The revised El Escorial (Airlie House) diagnostic criteria are criteria for the diagnosis of ALS, which have been widely accepted over the world. These diagnostic criteria are described in details as follows in "3. Grade Identification of Diagnostic Certainty, III. Diagnosis and/or Differential Diagnosis, ALS Therapy Guideline 2002, Treatment Guideline of the Societas Neurologica Japonica."

### 1) The revised El Escorial (Airlie House) diagnostic criteria

In 1990, Asociación Española de Esclerosis Lateral Amiotrófica (ADELA) divided the clinical diagnostic certainty of ALS into 4 stages by utilizing information obtained from electromyogram, nerve conduction speed and imaging findings, as well as the clinical findings as described below. The diagnostic criteria were further revised in 1994, and were widely accepted as EL Escorial diagnostic criteria of the World Federation of Neurology (WFN). Thereafter, in 1998, in order to increase the diagnostic sensitivity of El Escorial, the motor neuron disease study committee of the World Federation of Neurology gathered opinions under BR Brooks at the Airlie House Meeting (U.S.A.), and the following criteria were proposed. The understanding of these criteria is important for determining the degree of diagnostic certainty or for clinical studies such as determination of drug effects. These new criteria are different from the previous EL Escorial criteria. Specifically, a group, in which acute denervation findings are observed in at least two different muscles governed by nerve root or peripheral nerve, at which clinical disorders are not found, as well as clinical findings of upper and/or lower motor neuron degeneration in one body site, is added as "Probable ALS." That is, clinical findings such as electromyographical findings were permitted to be used instead of clinical findings such as muscular weakness and/or amyotrophia in a specific site, and the findings such as muscular weakness and amyotrophia were considered to be equivalent to electromyographically found degeneration of lower motor neurons. The lower motor neuron degeneration that is electromyographically defined by this method includes acute denervation findings (fibrillar contraction and positive sharp wave) and chronic denervation findings (high amplitudes and/or persistent motor units), and it also includes findings regarding a decrease in recruitment (an increase in motor units). The present diagnostic criteria were created for the purpose of increasing diagnostic sensitivity rather than the criteria of El Escorial for the need to incorporate early symptoms into therapeutic studies. However, there are also therapeutic studies based on other criteria or a critical eye on the present diagnostic criteria. In the Ireland ALS, Traynor et al. (2000) pointed out that the diagnostic sensitivity was never increased by the present diagnostic criteria, and they proposed the slowing of the criteria.

### 2) The points of the Airlie House diagnostic criteria are as follows:

1. signs of upper motor neuron lesion;
2. signs of lower motor neuron lesion; and
3. conditions deteriorated over at least 6-12 months after the onset of the disease.

When there are the following findings and such findings cannot be explained with diseases other than ALS, aging and the like, they conflict with the diagnosis of amyotrophic lateral sclerosis:
1. sensory disturbance;
2. sphincter disturbance;
3. autonomic disturbance;
4. forward visual field abnormality;
5. movement disorders found in Parkinson's disease; and
6. cognitive disorders found in Alzheimer's disease.

Before the ALS diagnostic criteria, the following A is present:
A: 1. evidence of lower motor neuron degeneration by clinical, electrophysiological or neuropathologic examination;
A: 2. evidence of upper motor neuron degeneration by clinical examination; and
A: 3. progressive spread of symptoms or signs within a region (e.g. right upper limb) or to other regions, as determined by history or nerve examination.

In addition to the above A, the absence of the following B is necessary:
B: 1. electrophysiological or pathological evidence of other disease processes that might explain the signs of upper and/or lower motor neuron degeneration; and
B: 2. neuroimaging evidence of other disease process that might explain the observed clinical and electrophysiological signs.

### Diagnostic Criteria (See the following table.)

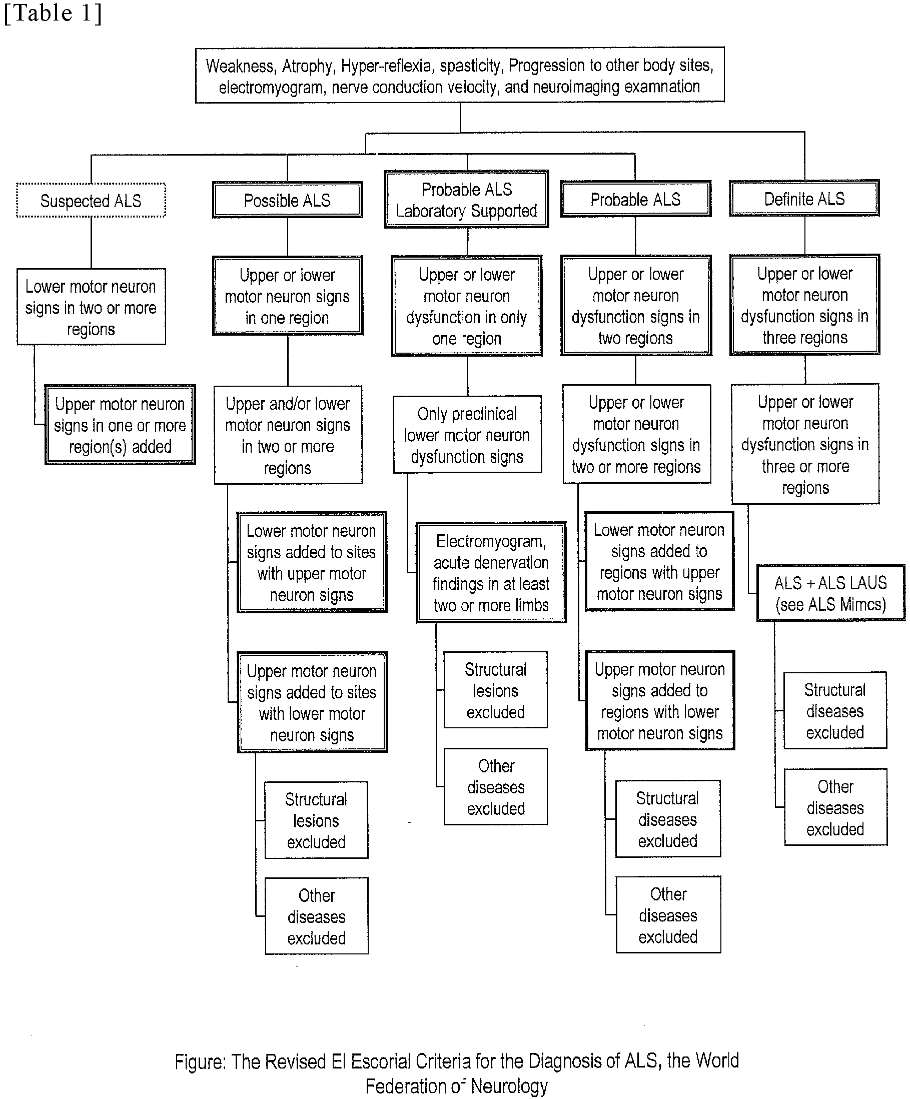

1. Clinically definite amyotrophic lateral sclerosis (clinically definite ALS, "Definite ALS") means that there are clinical signs of upper motor neuron or lower motor neuron lesion in three regions.
2. Clinically probable amyotrophic lateral sclerosis (clinically probable ALS, "Probable ALS") means that there are the findings of upper or lower motor neuron lesion in at least two regions, and that there also are the signs of upper motor neuron lesion closer to the head side than to the lower motor neuron lesion.
3. Clinically probable and laboratory-supported amyotrophic lateral sclerosis (clinically probable-laboratory-supported ALS, "Probable ALS laboratory-supported") means that the clinical signs of upper or lower motor neuron lesion are found only in one region, or that the signs of upper motor neuron lesion are found in one region, and the signs of lower motor neuron lesion are found in two or more limbs by needle electromyography, and further, other diseases can be eliminated by neuroimaging examinations or other examinations.
4. Clinically possible amyotrophic lateral sclerosis (clinically possible ALS, "Possible ALS") means that the signs of lower or upper motor neuron lesion are found in only one region, or that only the lower motor neuron lesion is found in two or more regions. Otherwise, the signs of the lower motor neuron lesion are found in a site closer to the head side than to the upper motor neuron lesion. The third clinically probable-laboratory supported amyotrophic lateral sclerosis (clinically probable-laboratory-supported ALS) is not satisfied with this definition. However, it is defined that other diseases can be eliminated.
5. Clinically suspected amyotrophic lateral sclerosis (clinically suspected ALS, "Suspected ALS") means that the signs of pure lower motor neuron lesion are found, and this is not suitable as a group used for the purpose of clinically studying amyotrophic lateral sclerosis. Accordingly, the clinically suspected ALS is eliminated from the Revised El Escorial Criteria for the Diagnosis of ALS, the World Federation of Neurology.

ALSFRS-R is the following evaluation scale. That is, ALSFRS-R is constituted with 12 items in total, which include limb movement disorder, bulbar dysfunction and the impairment of respiratory function. However, with regard to eating behavior, the relevant items are selected based on the presence or absence of gastrostomy, and then, the eating behavior is evaluated (Cranial Nerve 53(4) 346-355, April, 2001).

**[Table 2]**

| | |
|---|---|
| SPEECH | DRESSING AND HYGIENE |
| 4: Normal speech processes, | 4: Normal function, |
| 3: Detectable speech disturbance. | 3: Independent and complete self-care with effort or decreased efficiency. |
| 2: Intelligible with repeating, | |
| 1: Speech combined with nonvocal communication. | 2: Intermittent assistance or substitute methods. |
| 0: Loss of useful speech. | 1: Needs attendant for self-care. |
| SALIVATION | 0: Total dependence. |
| 4: Normal, | TURNING IN BED AND ADJUSTING BED CLOTHES |
| 3: Slight but definite excess of saliva in mouth; may have nighttime drooling. | 4: Normal. |
| | 3: Somewhat slow and clumsy, but no help needed. |
| 2: Moderately excessive saliva ; may have minimal drooling. | 2: Can turn alone or adjust sheets, but with great difficulty, |
| 1: Marked excess of saliva with some drooling. | 1: Can initiate, but not turn or adjust sheets alone. |
| 0: Marked drooling ; requires constant tissue or handkerchief, | 0: Helpless. |
| | WALKING |
| SWALLOWING | 4: Normal. |
| 4: Normal eating habits. | 3: Early ambulation difficulties. |
| 3: Early eating problems-occasional choking. | 2: Walks with assistance. |
| 2: Dietary consistency changes. | 1: Nonambulatory functional movement only. |
| 1: Needs supplemental tube feeding, | 0: No purposeful leg movement. |
| 0: NPO (exclusively parenteral or enteral feeding). | |
| | CLIMBING UPSTAIRS |
| HANDWRITING | 4: Normal. |
| 4: Normal. | 3: Slow. |
| 3: Slow or sloppy ; all words are legible. | 2: Mild unsteadiness or fatigue. |
| 2: Not all words are legible. | 1: Needs assistance. |
| 1: Able to grip pen but unable to write. | 0: Cannot do. |
| 0: Unable to grip pen. | DYSPNEA |
| CUTTING FOOD AND HANDLING UTENSILS | 4: None. |
| (patients without gastrostomy) | 3: Occurs when walking, |
| 4: Normal. | 2: Occurs with one or more of the following: eating, bathing, dressing (ADL). |
| 3: Somewhat slow and clumsy, but no help needed. | |
| 2: Can cut most foods, although clumsy and slow; some help needed, | 1: Occurs at rest, difficulty breathing when either sitting or lying, |
| 1: Food must be cut by someone, but can still feed slowly. | 0: Significant difficulty, considering using mechanical |
| 0: Needs to be fed. | respiratory support. |
| (alternate scale for patients with gastrostomy) | ORTHOPNEA |
| 4: Normal. | 4: None. |
| 3: Clumsy but able to perform all manipulations independently, | 3: Some difficulty sleeping at night due to shortness of breath, does not routinely use more than two pillows. |
| 2: Some help needed with closures and fasteners. | 2: Needs extra pillow in order to sleep (more than two), |
| 1: Provides minimal assistance to caregiver. | 1: Can only sleep sitting up. |
| 0: Unable to perform any aspect of task. | 0: Unable to sleep. |
| | RESPIRATORY INSUFFICIENCY |
| | 4: None. |
| | 3: Intermittent use of bipap. |
| | 2: Continuous use of bipap during the night, |
| | 1: Continuous use of bipap during the night and day, |
| | 0: Invasive mechanical ventilation by intubation or tracheostomy, |
| | |

The term "% FVC" is used herein to mean % predicted forced vital capacity (percent-predicted forced vital capacity), and it is calculated by the formula: the measurement value of forced vital capacity (FVC) / predicted vital capacity × 100. The predicted vital capacity is calculated based on sex, age and body height. In the case of a male, the calculation formula of the predicted vital capacity is (27.63 - 0.112 × age) × body height (cm), and in the case of a female, it is (21.78 - 0.101 × age) × body height (cm). In ALS, a %FVC value of 50% or less is defined as a standard for respiratory assistance (VIII. Respiratory Management and/or Nutritional Management, ALS Therapy Guideline 2002, Treatment Guideline of the Societas Neurologica Japonica). Moreover, since the normal value of % FVC is generally recognized to be 80% or more, the phrase "80% or more %FVC" is used in the present description to have the same meaning as "normal" or "almost normal" respiratory function.

Hereinafter, the present disclosure will be more specifically described in the following examples.

### [Examples]

### (Test Method)

199 patients with amyotrophic lateral sclerosis who complied with the following selection standards were divided into an actual drug administration group consisting of 100 patients and a placebo administration group of 99 patients:
1) patients who comply with any one of "Definite ALS" "Probable ALS," "Probable ALS-test positive," and "ALS possible" according to the revised El Escorial (Airlie House) diagnostic criteria;
2) patients who are able to have a meal, excrete and move by themselves, and do not need assistance in a daily life;
3) patients who are within 3 years after the onset of ALS;
4) patients who are between the age of 20 (inclusive) and 75 (inclusive);
5) patients who have a change in the ALSFRS-R scores of -1 to -4 points for 12 weeks before initiation of the administration;
6) patients who do not have respiratory difficulty due to a decrease in respiratory function; and
7) patients who do not have complications such as Parkinson's disease, schizophrenia or dementia, which may have influence on evaluation of drug effects.

With regard to the actual drug administration group, two ampules of "Radicut (registered trademark), Injection 30 mg" (containing 30 mg of 3-methyl-1-phenyl-2-pyrazolin-5-one; manufactured and distributed by Mitsubishi Tanabe Pharma Corporation) were diluted with a suitable amount of normal saline before the use, and the thus prepared solution was intravenously administered to the patients by drops over 60 minutes once a day. On the other hand, with regard to the placebo administration group, only normal saline was administered to the patients in the same manner as for the actual drug administration group.

The administration period was divided into first to sixth courses. The first course consisted of a continuous 14-day administration period and the subsequent drug holiday period for 14 days in which drug administration was not carried out. In the second to sixth courses, 1 set consisting of an administration period for a total of 10 out of 14 days and the subsequent 14-day drug holiday period was repeated 5 times. Drug administration is shown in the following table.

**[Table 3]**

| Table 3: Drug administration | | | | | |
|---|---|---|---|---|---|
| First course | | Second course | | Third course | |
| Administration period | Drug holiday period | Administration period | Drug holiday period | Administration period | Drug holiday period |
| 14 days | 14 days | Total of 10 out of 14 days | 14 days | Total of 10 out of 14 days | 14 days |

| Fourth course | | Fifth course | | Sixth course | |
|---|---|---|---|---|---|
| Administration period | Drug holiday period | Administration period | Drug holiday Period | Administration period | Drug holiday period |
| Total of 10 out of 14 days | 14 days | Total of 10 out of 14 days | 14 days | Total of 10 out of 14 days | 14 days |

### (Test Results)

Before initiation of the drug administration in the first course, and upon termination of the drug holiday periods in the first, second, third, fourth, fifth and sixth courses, the 199 patients were measured in terms of ALSFRS-R. Among the 199 patients, some patients were dropped out from the test during a period after initiation of the administration in the fourth course and before termination of the drug holiday period in the sixth course. In the case of these drop-out patients, the ALSFRS-R score measured immediately before dropping out from the test was considered to be the score upon termination of the drug holiday period in the sixth course, and it was used in the analysis.

A difference between the ALSFRS-R scores between before initiation of the administration in the first course and upon termination of the drug holiday period in the sixth course is shown below.

### [Table 4]

**Table 4:**

| | Number of patients | Before-and-after difference | Between-group difference | S. D. | t-test |
|---|---|---|---|---|---|
| Actual drug administration group | 100 | -5.3 | 0.7 | 5.4 | p>0.05 |
| Placebo administration group | 99 | -6.0 | | 6.0 | |

Subsequently, the 199 patients were divided into the following (1) to (7):
(1) patients who were determined to be "Definite ALS" or "Probable ALS" according to the revised El Escorial (Airlie House) diagnostic criteria;
(2) patient who were determined to be "Definite ALS" according to the revised El Escorial (Airlie House) diagnostic criteria;
(3) patients who scored two or more points from all items constituting the ALSFRS-R;
(4) patients whose %FVC was 80% or more;
(5) patients who scored two or more points from all items constituting the ALSFRS-R and whose %FVC was 80% or more (present invention);
(6) patients who were determined to be "Definite ALS" or "Probable ALS" according to the revised El Escorial (Airlie House) diagnostic criteria, scored two or more points from all items constituting the ALSFRS-R, and whose %FVC was 80% or more (present invention); and
(7) patients who were determined to be "Definite ALS" according to the revised El Escorial (Airlie House) diagnostic criteria, scored two or more points from all items constituting the ALSFRS-R, and whose %FVC was 80% or more. With regard to the thus specified patients (1) to (7), differences in the ALSFRS-R scores between before the administration in the first course and upon termination of the drug holiday period in the sixth course are shown below (present invention).

### [Table 5]

**Table 5**

| (1) Definite ALS or Probable ALS | | | | | |
|---|---|---|---|---|---|
| | Number of patients | Before-and-after difference | Between-group difference | S. D. | t-test |
| Actual drug administration group | 80 | -5.7 | 1.2 | 5.7 | p>0.05 |
| Placebo administration group | 71 | -6.9 | | 6.4 | |

### (2) Definite ALS

| | Number of patients | Before-and-after difference | Between-group difference | S. D. | t-test |
|---|---|---|---|---|---|
| Actual drug administration group | 28 | -6.7 | 2.0 | 6.7 | p>0.05 |
| Placebo administration group | 21 | -8.7 | | 7.8 | |

### (3) Two or more points from all items constituting ALSFRS-R

| | Number of patients | Before-and-after difference | Between-group difference | S. D. | t-test |
|---|---|---|---|---|---|
| Actual drug administration group | 64 | -4.7 | 1.6 | 5.6 | p>0.05 |
| Placebo administration group | 65 | -6.3 | | 6.4 | |

### (4) 80% or more %FVC

| | Number of patients | Before-and-after difference | Between-group difference | S. D. | t-test |
|---|---|---|---|---|---|
| Actual drug administration group | 79 | -3.9 | 1.3 | 3.6 | p>0.05 |
| Placebo administration group | 74 | -5.2 | | 5.9 | |

### (5) Two or more points from all items constituting ALSFRS-R and 80% or more %FVC (present invention)

| | Number of patients | Before-and-after difference | Between-group difference | S. D. | t-test |
|---|---|---|---|---|---|
| Actual drug administration group | 53 | -3.4 | 2.5 | 3.4 | p=0.01 84 |
| Placebo administration group | 46 | -5.9 | | 6.8 | |

### (6) Definite ALS or Probable ALS, two or more points from all items constituting ALSFRS-R, and 80% or more %FVC (present invention)

| | Number of patients | Before-and-after difference | Between-group difference | S. D. | t-test |
|---|---|---|---|---|---|
| Actual drug administration group | 45 | -3.8 | 3.7 | 3.4 | p=0.0038 |
| Placebo administration group | 32 | -7.5 | | 7.3 | |

### (7) Definite ALS, two or more points from all items constituting ALSFRS-R, and 80% or more %FVC (present invention)

| | Number of patients | Before-and-after difference | Between-group difference | S. D. | t-test |
|---|---|---|---|---|---|
| Actual drug administration group | 18 | -4.6 | 6.4 | 4.2 | p=0.0126 |
| Placebo administration group | 10 | -11.0 | | 8.6 | |

Even in a case in which patients with amyotrophic lateral sclerosis were not specified, while the ALSFRS-R score was decreased by 6.0 points in the placebo administration group, it was decreased by only 5.3 points in the actual drug administration group. Thus, it was found that 3-methyl-1-phenyl-2-pyrazolin-5-one exhibited effects on the treatment of amyotrophic lateral sclerosis or suppression of the disease progress thereof, or on the treatment of symptoms caused by amyotrophic lateral sclerosis or suppression of the disease progress thereof. However, it became clear that, even among such amyotrophic lateral sclerosis patients, the effects of 3-methyl-1-phenyl-2-pyrazolin-5-one were more clearly exhibited on the following ALS patients: (1) patients who are determined to be "Definite ALS" or "Probable ALS" according to the revised El Escorial (Airlie House) diagnostic criteria; (2) patient who are determined to be "Definite ALS" according to the revised El Escorial (Airlie House) diagnostic criteria; (3) patients who score two or more points from all items constituting the ALSFRS-R; (4) patients whose %FVC is 80% or more; (5) patients who score two or more points from all items constituting the ALSFRS-R and whose %FVC is 80% or more; (6) patients who are determined to be "Definite ALS" or "Probable ALS" according to the revised El Escorial (Airlie House) diagnostic criteria, score two or more points from all items constituting the ALSFRS-R, and whose %FVC is 80% or more; and (7) patients who are determined to be "Definite ALS" according to the revised El Escorial (Airlie House) diagnostic criteria, score two or more points from all items constituting the ALSFRS-R, and whose %FVC is 80% or more. That is to say, when patients with amyotrophic lateral sclerosis were not specified, a difference in the ALSFRS-R scores between before initiation of the administration in the first course and upon termination of the drug holiday period in the sixth course was -5.3 in the actual drug administration group, whereas the same difference in the ALSFRS-R scores was -6.0 in the placebo administration group. Thus, the between-group difference was 0.7. In contrast, in the case of (1) patients who are determined to be "Definite ALS" or "Probable ALS" according to the revised El Escorial (Airlie House) diagnostic criteria, the between-group difference was 1.2; in the case of (2) patient who are determined to be "Definite ALS" according to the revised El Escorial (Airlie House) diagnostic criteria, the between-group difference was 2.0; in the case of (3) patients who score two or more points from all items constituting the ALSFRS-R, the between-group difference was 1.6; in the case of (4) patients whose %FVC is 80% or more, the between-group difference was 1.3; in the case of (5) patients who score two or more points from all items constituting the ALSFRS-R and whose %FVC is 80% or more, the between-group difference was 2.5; in the case of (6) patients who are determined to be "Definite ALS" or "Probable ALS" according to the revised El Escorial (Airlie House) diagnostic criteria, score two or more points from all items constituting the ALSFRS-R, and whose %FVC is 80% or more, the between-group difference was 3.7; and in the case of (7) patients who are determined to be "Definite ALS" according to the revised El Escorial (Airlie House) diagnostic criteria, score two or more points from all items constituting the ALSFRS-R, and whose %FVC is 80% or more, the between-group difference was 6.4. Hence, all of the obtained values were greater than the aforementioned value 0.7. Even in the case of (1), which had the smallest between-group difference value among the aforementioned (1) to (7), the value is 1.2. This value has a difference of 0.5 from the value obtained in the case of not specifying patients with amyotrophic lateral sclerosis (0.7). It has been reported that a difference of 1 point in the ALSFRS-R score would increase the risk of death or tracheostomy for patients with amyotrophic lateral sclerosis by 7% (Neurology 2005; 64: 38-43). Accordingly, in the case of specific patients in the present disclosure, the risk of such death or tracheostomy can be reduced by 3.5% or more, and thus, this is greatly advantageous for patients with amyotrophic lateral sclerosis. Further, the between-group differences were 2.5, 3.7 and 6.4 in the patient groups (5) to (7) above, respectively. Thus, excellent results could be obtained, in which these values were 3 times or more greater than the between-group difference obtained in the case of not specifying patient groups (0.7). It was also found that these patient groups (5) to (7) each had a significant difference, in comparison with the placebo administration group.

## Claims

1. A pharmaceutical agent for use in treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein a patient administered with the agent is a patient
[1] who scores two or more points from all items constituting the ALSFRS-R; and
[2] whose %FVC is 80% or more,
and wherein the applied dose of 3-methyl-I-phenyl-2-pyrazolin-5-one or a physiologically acceptable salt thereof per day corresponds to an amount of 15 mg to 240 mg 3-methyl-1-phenyl-2-pyrazolin-5-one.

2. A pharmaceutical agent for use in treating amyotrophic lateral sclerosis or suppressing the disease progress thereof, or treating symptoms caused by amyotrophic lateral sclerosis or suppressing the disease progress thereof, which comprises, as an active ingredient, 3-methyl-1-phenyl-2-pyrazolin-5-one or a physiologically acceptable salt thereof, wherein the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period, and wherein the agent is administered to a patient with amyotrophic lateral sclerosis, and the amyotrophic lateral sclerosis of the patient administered with the agent is amyotrophic lateral sclerosis
[1] which scores two or more points from all items constituting the ALSFRS-R; and
[2] whose %FVC is 80% or more
and wherein the applied dose of 3-methyl-I-phenyl-2-pyrazolin-5-one or a physiologically acceptable salt thereof per day corresponds to an amount of 15 mg to 240 mg 3-methyl-1-phenyl-2-pyrazolin-5-one.

3. The pharmaceutical agent for use according to claim 1 or 2, wherein with regard to the administration period and the drug holiday period, an initial 14-day administration period and an initial 14-day drug holiday period are established, and thereafter, an administration period for 10 out of 14 days and a 14-day drug holiday period are repeated.

4. The pharmaceutical agent for use according to any one of claims 1 to 3, wherein the symptoms caused by amyotrophic lateral sclerosis are a decrease in respiratory function, spoken language disorder, dysphagia, or limb movement disorder.

## Patentansprüche

1. Pharmazeutischer Wirkstoff zur Verwendung bei der Behandlung von amyotropher Lateralsklerose oder der Hemmung des Fortschreitens der Krankheit oder bei der Behandlung von Symptomen, die durch die amyotrophe Lateralsklerose verursacht werden, oder der Hemmung des Fortschreitens der Krankheit, der als Wirkstoff 3-Methyl-1-phenyl-2-pyrazolin-5-on oder ein physiologisch akzeptables Salz davon umfasst, wobei der Wirkstoff verabreicht wird, indem ein 14-tägiger Verabreichungszeitraum und ein 14-tägiger Medikamentenpausenzeitraum wiederholt werden, oder indem ein anfänglicher 14-tägiger Verabreichungszeitraum und ein anfänglicher 14-tägiger Medikamentenpausenzeitraum festgelegt werden, gefolgt von einer Wiederholung des Verabreichungszeitraums von 10 von 14 Tagen und eines 14-tägigen Medikamentenpausenzeitraums, und wobei ein Patient, der mit dem Wirkstoff behandelt wird, ein Patient ist
[1] der aus allen Elementen, die den ALSFRS-R bilden, zwei oder mehr Punkte erhält; und
[2] dessen %FVC 80 % oder mehr beträgt,
und wobei die angewendete Dosis von 3-Methyl-1-phenyl-2-pyrazolin-5-on oder einem physiologisch akzeptablen Salz davon pro Tag einer Menge von 15 mg bis 240 mg 3-Methyl-1-phenyl-2-pyrazolin-5-on entspricht.

2. Pharmazeutischer Wirkstoff zur Verwendung bei der Behandlung von amyotropher Lateralsklerose oder der Hemmung des Fortschreitens der Krankheit oder der Behandlung von Symptomen, die durch die amyotrophe Lateralsklerose verursacht werden, oder der Hemmung des Fortschreitens der Krankheit, der als Wirkstoff 3-Methyl-1-phenyl-2-pyrazolin-5-on oder ein physiologisch akzeptables Salz davon umfasst, wobei der Wirkstoff verabreicht wird, indem ein 14-tägiger Verabreichungszeitraum und ein 14-tägiger Medikamentenpausenzeitraum wiederholt werden, oder indem ein anfänglicher 14-tägiger Verabreichungszeitraum und ein anfänglicher 14-tägiger Medikamentenpausenzeitraum festgelegt werden, gefolgt von einer Wiederholung des Verabreichungszeitraums für 10 von 14 Tagen und eines 14-tägigen Medikamentenpausenzeitraums, und wobei der Wirkstoff einem Patienten mit amyotropher lateraler Sklerose verabreicht wird und die amyotrophe laterale Sklerose des mit dem Wirkstoff behandelten Patienten ebenfalls amyotrophe laterale Sklerose ist
[1] die aus allen Elementen, die den ALSFRS-R bilden, zwei oder mehr Punkte erhält; und
[2] dessen %FVC 80 % oder mehr beträgt
und wobei die angewendete Dosis von 3-Methyl-1-phenyl-2-pyrazolin-5-on oder einem physiologisch akzeptablen Salz davon pro Tag einer Menge von 15 mg bis 240 mg 3-Methyl-1-phenyl-2-pyrazolin-5-on entspricht.

3. Pharmazeutischer Wirkstoff zur Verwendung nach Anspruch 1 oder 2, wobei in Bezug auf den Verabreichungszeitraum und den Medikamentenpausenzeitraum ein anfänglicher 14-tägiger Verabreichungszeitraum und ein anfänglicher 14-tägiger Medikamentenpausenzeitraum festgelegt werden, und danach ein Verabreichungszeitraum von 10 von 14 Tagen sowie ein 14-tägiger Medikamentenpausenzeitraum wiederholt werden.

4. Pharmazeutischer Wirkstoff zur Verwendung nach einem der Ansprüche 1 bis 3, wobei die Symptome, die durch amyotrophe laterale Sklerose verursacht werden, eine Abnahme der Atemfunktion, eine Störung der gesprochenen Sprache, Dysphagie oder eine Störung der Bewegungen der Gliedmaßen umfassen.

## Revendications

1. Agent pharmaceutique pour une utilisation dans le traitement de la sclérose latérale amyotrophique ou une suppression de son évolution, ou un traitement de symptômes causés par la sclérose latérale amyotrophique ou une suppression de leur évolution, qui comprend, en tant qu'ingrédient actif, de la 3-méthyl-1-phényl-2-pyrazolin-5-one ou un sel physiologiquement acceptable de celle-ci, dans lequel l'agent est administré en répétant une période d'administration de 14 jours et une période de pause médicamenteuse de 14 jours, ou en établissant une période initiale d'administration de 14 jours et une période initiale de pause médicamenteuse de 14 jours, puis en répétant une période d'administration de 10 jours sur 14 et une période de pause médicamenteuse de 14 jours, et dans lequel un patient auquel est administré l'agent est un patient
[1] qui obtient deux points ou plus pour tous les éléments constituant l'ALSFRS-R ; et
[2] dont le % de CVF est de 80 % ou plus,
et dans lequel la dose appliquée de 3-méthyl-1-phényl-2-pyrazolin-5-one ou d'un sel physiologiquement acceptable de celle-ci par jour correspond à une quantité de 15 mg à 240 mg de 3-méthyl-1-phényl-2-pyrazolin-5-one.

2. Agent pharmaceutique pour une utilisation dans le traitement de la sclérose latérale amyotrophique ou une suppression de son évolution, ou un traitement de symptômes causés par la sclérose latérale amyotrophique ou une suppression de leur évolution, qui comprend, en tant qu'ingrédient actif, de la 3-méthyl-1-phényl-2-pyrazolin-5-one ou un sel physiologiquement acceptable de celle-ci, dans lequel l'agent est administré en répétant une période d'administration de 14 jours et une période de pause médicamenteuse de 14 jours, ou en établissant une période initiale d'administration de 14 jours et une période initiale de pause médicamenteuse de 14 jours, puis en répétant une période d'administration de 10 jours sur 14 et une période de pause médicamenteuse de 14 jours, et dans lequel l'agent est administré à un patient atteint de sclérose latérale amyotrophique, et la sclérose latérale amyotrophique du patient auquel est administré l'agent est une sclérose latérale amyotrophique
[1] qui obtient deux points ou plus pour tous les éléments constituant l'ALSFRS-R ; et
[2] dont le % de CVF est de 80 % ou plus
et dans lequel la dose appliquée de 3-méthyl-1-phényl-2-pyrazolin-5-one ou d'un sel physiologiquement acceptable de celle-ci par jour correspond à une quantité de 15 mg à 240 mg de 3-méthyl-1-phényl-2-pyrazolin-5-one.

3. Agent pharmaceutique pour une utilisation selon la revendication 1 ou la revendication 2, dans lequel, en ce qui concerne la période d'administration et la période de pause médicamenteuse, une période initiale d'administration de 14 jours et une période initiale de pause médicamenteuse de 14 jours sont établies, et par la suite, une période d'administration de 10 jours sur 14 et une période de pause médicamenteuse de 14 jours sont répétées.

4. Agent pharmaceutique pour une utilisation selon l'une quelconque des revendications 1 à 3, dans lequel les symptômes causés par la sclérose latérale amyotrophique sont une diminution de fonction respiratoire, un trouble du langage parlé, une dysphagie ou un trouble de mouvements des membres.
